(19) Europäisches Patentamt
European Patent Office
Office européen des brevets

(11) **EP 2 260 756 A1**

(12) **EUROPÄISCHE PATENTANMELDUNG**

(43) Veröffentlichungstag:
**15.12.2010 Patentblatt 2010/50**

(51) Int Cl.:
*A61B 5/00* (2006.01)          *A61B 5/053* (2006.01)

(21) Anmeldenummer: **10009799.7**

(22) Anmeldetag: **09.08.2006**

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **09.08.2005 DE 102005038035**
**09.09.2005 DE 102005043606**
**25.10.2005 DE 102005051030**

(62) Dokumentnummer(n) der früheren Anmeldung(en) nach Art. 76 EPÜ:
**06776705.3 / 1 931 239**

(71) Anmelder: **Flore, Ingo**
**44141 Dortmund (DE)**

(72) Erfinder: **Die Erfindernennung liegt noch nicht vor**

(74) Vertreter: **Isfort, Olaf**
**Schneiders & Behrendt,**
**Huestrasse 23**
**44787 Bochum (DE)**

Bemerkungen:
Diese Anmeldung ist am 17-09-2010 als Teilanmeldung zu der unter INID-Code 62 erwähnten Anmeldung eingereicht worden.

(54) **Medizinische Messvorrichtung**

(57)    Die Erfindung betrifft eine Messvorrichtung (1) zur nicht-invasiven Bestimmung von physiologischen Parametern, mit wenigstens einer optischen Messeinheit (100) zur Erzeugung von oximetrischen und/oder plethysmographischen Messsignalen, und mit einer Auswertungseinheit (140) zur Verarbeitung der Messsignale, wobei die Auswertungseinheit (140) eingerichtet ist zur Bestimmung wenigstens eines lokalen metabolischen Parameters aus den Signalen der optischen Messeinheit (100). Die Erfindung schlägt vor, dass die optische Messeinheit (100) wenigstens zwei Strahlungsquellen (4, 4') zur Bestrahlung des untersuchten Körpergewebes (240), und wenigstens einen Strahlungssensor (5) zur Detektion der von dem Körpergewebe (240) gestreuten und/oder transmittierten Strahlung aufweist, wobei die Strahlungsquellen (4, 4') unterschiedliche Volumenbereiche des untersuchten Körpergewebes (240) bestrahlen und hierzu unterschiedliche räumliche Abstrahlcharakteristiken haben, wobei die Auswertungseinheit (140) eingerichtet ist zur Bestimmung des lokalen Sauerstoffverbrauchs und/oder des Blutglukosespiegels anhand der Intensitäten der von dem Körpergewebe (240) gestreuten und/oder transmittierten Strahlung der beiden Strahlungsquellen (4, 4').

Fig. 8

**Beschreibung**

Die Erfindung betrifft eine Messvorrichtung zur nicht-invasiven Bestimmung von physiologischen Parametern, mit wenigstens einer optischen Messeinheit zur Erzeugung von oximetrischen und/oder plethysmographischen Messsignalen, und mit einer Auswertungseinheit zur Verarbeitung der Messsignale, wobei die Auswertungseinheit eingerichtet ist zur Bestimmung wenigstens eines lokalen metabolischen Parameters aus den Signalen der optischen Messeinheit.

Außerdem betrifft die Erfindung ein Verfahren zur Erfassung und Auswertung von physiologischen Parametern, wobei mittels einer optischen Messeinheit oximetrische und/oder plethysmographische Messsignale von Körpergewebe erfasst werden und mittels einer Auswertungseinheit die oximetrischen und/oder plethysmographischen Messsignale verarbeitet werden, und zwar zur Bestimmung des Pulses und/oder der lokalen Sauerstoffkonzentration, wobei mittels der Auswertungseinheit wenigstens ein lokaler metabolischer Parameter aus den oximetrischen Signalen bestimmt wird.

Die Versorgung des Körpergewebes mit Sauerstoff gehört bekanntlich zu den wichtigsten Vitalfunktionen des Menschen. Aus diesem Grund sind oximetrische Diagnosemodalitäten heutzutage von großer Bedeutung in der Medizin. Routinemäßig werden sogenannte Pulsoximeter eingesetzt. Derartige Pulsoximeter umfassen typischerweise zwei Lichtquellen, die rotes bzw. infrarotes Licht unterschiedlicher Wellenlänge in das Körpergewebe einstrahlen. Das Licht wird im Körpergewebe gestreut und teilweise absorbiert. Das gestreute Licht wird schließlich mittels eines Lichtsensors in Form einer geeigneten Photozelle detektiert. Typischerweise verwenden kommerzielle Pulsoximeter zum einen Licht im Wellenlängenbereich von 660 nm. In diesem Bereich ist die Lichtabsorption von Oxihämoglobin und Desoxihämoglobin stark unterschiedlich. Dementsprechend variiert die Intensität des mittels des Photosensors detektierten, gestreuten Lichts in Abhängigkeit davon, wie stark das untersuchte Körpergewebe von sauerstoffreichem, bzw. sauerstoffarmem Blut durchblutet ist. Zum anderen wird üblicherweise Licht im Wellenlängenbereich von 810 nm verwendet. Diese Lichtwellenlänge liegt im sogenannten nahen infraroten Spektralbereich. Die Lichtabsorption von Oxihämoglobin und Desoxihämoglobin ist in diesem Spektralbereich im Wesentlichen gleich. Die bekannten Pulsoximeter sind außerdem in der Lage, ein plethysmographisches Signal, d. h. ein Volumenpulssignal zu erzeugen, das die während des Herzschlags veränderliche Blutmenge in dem von dem Pulsoximeter erfassten Mikrogefäßsystem wiedergibt (sog. Photoplethysmographie). Bei Verwendung unterschiedlicher Lichtwellenlängen in den oben erwähnten Spektralbereichen kann aus der unterschiedlichen Lichtabsorption auf den Sauerstoffgehalt des Blutes (Sauerstoffsättigung) zurückgeschlossen werden. Die üblichen Pulsoximeter werden entweder an der Fingerspitze eines Patienten oder auch am Ohrläppchen eingesetzt. Es wird dann das Volumenpulssignal aus der Blutperfusion des Mikrogefäßsystems in diesen Bereichen des Körpergewebes erzeugt.

Aus der WO 00/69328 A1 ist ein besonders flexibel einsetzbares oximetrisches Diagnosegerät bekannt. Dieses vorbekannte Gerät ist handführbar ausgebildet, so dass es an beliebigen Messorten am menschlichen Körper eingesetzt werden kann. Das vorbekannte Gerät erlaubt gleichsam ein systematisches Abtasten ("Scannen") des Körpers eines Patienten. Eine Fixierung des Diagnosegerätes - wie bei üblichen Pulsoximetern - kann bei dem aus der genannten Druckschrift bekannten Gerät entfallen.

Die genannte WO 00/69328 A1 spricht außerdem die Einsetzbarkeit des oximetrischen Diagnosegerätes zur ortsaufgelösten Erkennung von Entzündungen, Tumoren und Arterioskleroseerkrankungen im hautoberflächennahen Körpergewebe eines Patienten an. Derartige Erkrankungen bewirken eine Veränderung der Durchblutung des Körpergewebes. Durch die ortsaufgelöste oximetrische Abtastung des Körpers lassen sich mit dem vorbekannten Gerät Veränderungen der Durchblutung, die auf eine entsprechende Erkrankung hindeuten, erkennen und lokalisieren.

Das EKG (Elektrokardiogramm) dürfte die am meisten eingesetzte Untersuchungsmodalität zur Diagnose von Herz-Kreislauf-Erkrankungen sein. Mittels eines EKG-Gerätes werden mit zwei oder mehr EKG-Elektroden elektrische Signale von dem Körper des zu untersuchenden Patienten abgeleitet. Das so gewonnene EKG gibt die bioelektrischen Spannungen, die bei der Erregungsausbreitung und -rückbildung am Herzen entstehen, wieder. Das EKG enthält zahlreiche diagnostisch auswertbare Parameter. Zum Zeitpunkt der Kontraktion des Herzmuskels während eines Herzschlags zeigt das EKG eine deutliche Spitze, die auch als R-Zacke bezeichnet wird. Weiterhin enthält das EKG die der R-Zacke vorangehende, so genannte P-Welle. Der R-Zacke folgt wiederum die so genannte T-Welle. Die Minima im EKG unmittelbar vor und unmittelbar nach der R-Zacke werden mit Q bzw. S bezeichnet. Für die Herz-Kreislauf-Diagnostik interessante Parameter sind die Dauer der P-Welle sowie die Amplitude der P-Welle, die Dauer des PQ-Intervalls, die Dauer des QRS-Komplexes, die Dauer des QT-Intervalls sowie die Amplitude der T-Welle. Sowohl aus den Absolutwerten der genannten Parameter wie auch aus den Verhältnissen der Parameter kann auf den Gesundheitszustand des Herz-Kreislauf-Systems geschlossen werden. Vorrichtungen und Verfahren zur EKG-Messung sind beispielsweise aus den Druckschriften US 6,331,162 oder US 4,960,126 vorbekannt.

Zur Bestimmung von weiteren physiologischen Parametern, wie z. B. Körperfettgehalt, ist das Prinzip der bioelektrischen Impedanzmessung beispielsweise aus der US 6,714,814 bekannt. Die Zusammensetzung des Körpergewebes kann aber auch optisch bestimmt werden. Das Prinzip der optischen Bestimmung des Körperfettgehalts mittels Infrarotlicht ist beispielsweise in der US 4,928,014 beschrieben.

Der vorliegenden Erfindung liegt die Aufgabe zugrunde, eine Vorrichtung zur nicht-invasiven Bestimmung von physiologischen Parametern bereit zu stellen, die gegenüber dem Stand der Technik verbessert und hinsichtlich ihrer Funktionalität erweitert ist.

Diese Aufgabe löst die Erfindung durch eine Messvorrichtung gemäß Patentanspruch 1 sowie durch ein Verfahren gemäß Patentanspruch 8.

Bevorzugte Ausgestaltungen der Erfindung sind Gegenstand der Unteransprüche.

Kerngedanke der Erfindung ist es, die mittels der optischen Messeinheit gewonnenen oximetrischen und/oder plethysmographischen Messsignale heranzuziehen, um nicht nur - wie z. B. bei den bekannten oximetrischen Diagnosegeräten - die lokale Sauerstoffkonzentration am jeweiligen Messort, sondern insbesondere auch den lokalen Sauerstoffverbrauch als wichtigen Indikator für die lokale metabolische Aktivität zu bestimmen. Erkrankungen können mit der erfindungsgemäßen Messvorrichtung anhand von pathologischen Veränderungen des Metabolismus erkannt und lokalisiert werden.

Die optische Messeinheit der erfindungsgemäßen Messvorrichtung arbeitet auf der Basis von optischen Messverfahren. Hierzu umfasst die Vorrichtung die Strahlungsquellen zur Bestrahlung des untersuchten Körpergewebes mit elektromagnetischer Strahlung, und wenigstens einen Strahlungssensor zur Bestimmung der von dem Körpergewebe gestreuten und/oder transmittierten elektromagnetischen Strahlung. Als Strahlungsquellen kommen übliche Leuchtdioden oder auch Laserdioden in Frage, die optische Strahlung, d.h. Licht im entsprechenden Spektralbereich emittieren. Als besonders vorteilhaft hat es sich erwiesen, wenn mit der erfindungsgemäßen Vorrichtung die Strahlungsabsorption im untersuchten Körpergewebe bei mindestens drei unterschiedlichen Lichtwellenlängen gemessen wird, um daraus die Sauerstoffkonzentration des Blutes und die Durchblutung des Gewebes zu bestimmen.

Gemäß einer sinnvollen Ausgestaltung weist die optische Messeinheit der erfindungsgemäßen Messvorrichtung wenigstens zwei Strahlungssensoren zur Detektion der von dem Körpergewebe gestreuten und/oder transmittierten Strahlung auf, wobei die Strahlungssensoren in unterschiedlichem Abstand zur Strahlungsquelle angeordnet sind. Dies eröffnet die Möglichkeit, Rückschlüsse auf die jeweils im Körpergewebe von der Strahlung zurückgelegte Strecke zu ziehen. Auf dieser Basis kann die Sauerstoffkonzentration im Blut und im Gewebe in unterschiedlich tiefen Gewebeschichten untersucht werden. Dabei kann ausgenutzt werden, dass die Messsignale aus den tiefer liegenden Gewebeschichten stärker vom arteriellen Blut beeinflusst sind, während in den oberflächennäheren Regionen die Strahlungsabsorption stärker von dem Blut im kapillaren Gefäßsystem beeinflusst ist.

Gemäß der Erfindung sind wenigstens zwei Strahlungsquellen vorgesehen, welche unterschiedliche Volumenbereiche des untersuchten Körpergewebes bestrahlen. Hierdurch lässt sich eine differenzielle Messung der Lichtabsorption einfach realisieren. Dies ermöglicht es, Metabolismus-induzierte Änderungen der Durchblutung des untersuchten Körpergewebes mit sauerstoffreichem bzw. sauerstoffarmem Blut zu untersuchen. Dabei wird ausgenutzt, dass sich in Abhängigkeit von der metabolischen Aktivität des Gewebes der lokale Sauerstoffverbrauch verändert. Die Bestimmung des veränderlichen Sauerstoffverbrauchs erlaubt wiederum Rückschlüsse auf den lokalen Energieverbrauch, der mit dem Sauerstoffverbrauch direkt korreliert ist. Besonders interessant ist, dass dies wiederum Rückschlüsse auf den Glukosespiegel zulässt. Somit erlaubt die erfindungsgemäße Messvorrichtung vorteilhafterweise auch eine nicht-invasive Bestimmung des Blutglukosespiegels.

Die zwei Strahlungsquellen der optischen Messeinheit der erfindungsgemäßen Messvorrichtung sind so ausgelegt, dass die von diesen jeweils bestrahlten Volumenbereiche hinsichtlich der Durchblutung mit sauerstoffarmem bzw. sauerstoffreichem Blut unterschiedlich betroffen sind. Dies wird dadurch erreicht, dass die wenigstens zwei Strahlungsquellen unterschiedliche räumliche Abstrahlcharakteristiken haben. So können als Strahlungsquellen z. B. eine Leuchtdiode und ein Laser verwendet werden, die ähnliche Wellenlängen (z. B. 630 nm und 650 nm) haben. Die beiden Strahlungsquellen unterscheiden sich aber durch den Öffnungswinkel der Abstrahlung. Während z. B. die Leuchtdiode unter einem großen Öffnungswinkel in das untersuchte Körpergewebe einstrahlt, tritt das Licht der Laserdiode unter einem sehr kleinen Öffnungswinkel in das Körpergewebe ein. Dies hat zur Folge, dass mit den beiden Strahlungsquellen unterschiedliche Volumenbereiche des Körpergewebes erfasst werden. Aufgrund des großen Öffnungswinkels wird von der Leuchtdiode ein größerer Volumenbereich der nicht-durchbluteten Epidermis erfasst als von dem Laser. Die undurchblutete Epidermis ist von einer Änderung der Hämoglobinkonzentration praktisch nicht betroffen. Dementsprechend ist die Intensität der von dem Körpergewebe gestreuten und/oder transmittierten Strahlung der Leuchtdiode weniger stark von einer Änderung der Hämoglobinkonzentration abhängig als die Intensität der Strahlung des Lasers. Voraussetzung ist, dass die Wellenlänge der von den beiden Strahlungsquellen jeweils emittierten Strahlung so gewählt wird, dass die Strahlung unterschiedlich stark durch Oxihämoglobin bzw. Desoxihämoglobin absorbiert wird. Die Wellenlänge sollte daher zwischen 600 und 700 nm, vorzugsweise zwischen 630 und 650 nm liegen.

Die Auswertungseinheit der erfindungsgemäßen Messvorrichtung ist zur Bestimmung des wenigstens einen lokalen metabolischen Parameters aus der von dem Körpergewebe gestreuten und/oder transmittierten Strahlung der beiden Strahlungsquellen ausgebildet. Wenn in dem untersuchten Körpergewebe Sauerstoff verbraucht wird, wird Oxihämoglobin in Desoxihämoglobin umgewandelt. Durch einen Vergleich der aus den unterschiedlichen Volumenbereichen des Körpergewebes stammenden Strahlung der beiden Strahlungsquellen kann die Änderung des Konzentrationsverhältnisses von Oxihämoglobin und Desoxihämoglobin festgestellt werden. Hieraus ergibt sich wiederum der lokale Sauerstoffverbrauch und daraus letztlich der Blutglukosespiegel. Somit ist die Auswertungseinheit der erfindungsgemäßen Messvorrichtung eingerichtet zur Bestimmung des lokalen Sauerstoffverbrauchs und/oder des Blutglukosespiegels anhand der Intensitäten der von dem Körpergewebe gestreuten und/oder transmittierten Strahlung der beiden Strahlungsquellen.

Gemäß einer bevorzugten Ausgestaltung weist die erfindungsgemäße Messvorrichtung zusätzlich eine Einheit zur Erfassung von lokalen Gewebeparametern, wie Fettgehalt, Wassergehalt und/oder Durchblutung auf, wobei die Auswertungseinheit in diesem Fall eingerichtet ist zur Bestimmung des wenigstens einen lokalischen metabolischen Parameters aus den Signalen der optischen Messeinheit und den Gewebeparametern.

Ein wichtiger lokaler Gewebeparameter im Sinne der Erfindung ist beispielsweise die Durchblutung. Damit sind die durchblutungsbedingten Volumenschwankungen des untersuchten Körpergewebes gemeint. Zur Erfassung der Durchblutung kann die erfindungsgemäße Messvorrichtung insofern mit einer Plethysmographieeinheit herkömmlicher Art (z. B. Photoplethysmograph) ausgestattet sein.

Die Erfindung basiert u. a. auf der Erkenntnis, dass durch die Kombination der Erfassung von oximetrischen und plethysmographischen Signalen die Möglichkeit eröffnet wird, lokale metabolische Parameter zu bestimmen.

Für die Ermittlung des lokalen Sauerstoffverbrauchs sollte mittels der erfindungsgemäßen Messvorrichtung zusätzlich zur oximetrisch bestimmten arteriellen Sauerstoffkonzentration auch die kapillare Sauerstoffkonzentration im Gewebe bestimmt werden können. Wichtige Parameter sind der lokale Fettgehalt und/oder der Wassergehalt des Körpergewebes. Diese Parameter können beispielsweise mittels bioelektrischer Impedanzmessung erfasst werden. Gemäß einer sinnvollen Ausgestaltung der Erfindung ist eine (optische) Oximetrieeinheit mit einer bioelektrischen Impedanzmesseinheit in einem einzigen Gerät kombiniert. Aus den mittels der bioelektrischen Impedanzmesseinheit gewonnenen Messsignalen kann die Zusammensetzung des untersuchten Körpergewebes bestimmt werden. Auf dieser Grundlage kann dann aus den oximetrischen Signalen mittels der Auswertungseinheit der Messvorrichtung die kapillare Sauerstoffsättigung im Gewebe ermittelt werden.

Eine sinnvolle Weiterbildung der erfindungsgemäßen Messvorrichtung sieht vor, dass die bioelektrische Impedanzmesseinheit außerdem zur Erfassung von globalen Gewebeparametern, wie globaler Fettgehalt und/oder globaler Wassergehalt, ausgebildet ist. Hierdurch wird die Funktionalität der erfindungsgemäßen Messvorrichtung erweitert. Die bioelektrische Impedanzmesseinheit der erfindungsgemäßen Messvorrichtung kann derart ausgestaltet sein, dass damit sowohl lokale wie auch globale Gewebeparameter gemessen werden können. Die Zusammensetzung des Körpergewebes kann mit der erfindungsgemäßen Messvorrichtung optisch bestimmt werden. Hierzu weist die Einheit zur Erfassung von lokalen Gewebeparametern eine optische Strahlungsquelle und einen Photosensor auf. Das Prinzip der optischen Bestimmung des Körperfettgehalts mittels Infrarotlicht ist aus dem Stand der Technik bekannt.

Gemäß einer vorteilhaften Ausgestaltung umfasst die erfindungsgemäße Vorrichtung einen Wärmesensor zur Bestimmung der lokalen Wärmeproduktion, wobei die Auswertungseinheit zur Bestimmung der lokalen metabolischen Parameter unter Berücksichtigung der Signale des Wärmesensors eingerichtet ist. Vorzugsweise ist mittels des Wärmesensors eine orts-, zeit- und tiefenaufgelöste Wärmemessung am Messort möglich. Anhand des Wärmeaustauschs kann auf die lokale Stoffwechselaktivität zurückgeschlossen werden. Außerdem ist der Wärmesensor zur Bestimmung der lokalen Durchblutung geeignet. Bezüglich näherer Hintergrundinformationen zur Wärmemessung wird auf die Veröffentlichung von Nitzan et al. verwiesen (Meir Nitzan, Boris Khanokh, "Infrared Radiometry of Thermally Insulated Skin for the Assessment of Skin Blood Flow", Optical Engineering 33, 1994, No. 9, S. 2953 bis 2956). Insgesamt liefert der Wärmesensor Daten, die mit Vorteil zur Bestimmung von metabolischen Parametern im Sinne der Erfindung genutzt werden können.

Die arterielle Sauerstoffsättigung ($SaO_2$) und die venöse Sauerstoffsättigung ($SvO_2$) bestimmen abhängig von der Art des untersuchten Gewebes die kapillare (arteriovenöse) Sauerstoffsättigung ($StO_2$). Es gilt:

$$K * SvO_2 + (1 - K) * SaO_2 = StO_2,$$

wobei K ein gewebeabhängiger Korrekturfaktor ist, der vom Volumenverhältnis von Arterien zu Venen im untersuchten Gewebe abhängt. Im Mittel liegt dieser Wert etwas unter 0,5. Der für das jeweilige Gewebe maßgebliche Wert kann gemäß der Erfindung durch bioelektrische Impedanzmessung ermittelt werden, um dann aus der obigen Formel die venöse Sauerstoffsättigung zu bestimmen. Mittels Wärmemessung und/oder bioelektrischer Impedanz (Impedanzplethysmographie) kann die Durchblutung V, d. h. die durchblutungsbedingte Volumenschwankung des Gewebes bestimmt werden. Nach der Beziehung

$$VO_2 = V * (SaO_2 - SvO_2)$$

kann dann schließlich der lokale Sauerstoffverbrauch VO$_2$ berechnet werden, der ein Maß für die metabolische Aktivität am Messort darstellt.

Des Weiteren kann die erfindungsgemäße Messvorrichtung einen optischen Sensor zur ortsaufgelösten Bestimmung des Hautkolorits umfassen. Auch anhand von lokalen Verfärbungen der Haut können Erkrankungen, wie zum Beispiel Entzündungen, Melanome usw., detektiert werden.

Durch eine zusätzliche EKG-Einheit zur Erfassung eines EKG-Signals über zwei oder mehr EKG-Elektroden wird der Funktionsumfang der erfindungsgemäßen Messvorrichtung vorteilhaft erweitert. Gemäß der Erfindung können mittels der Messvorrichtung plethysmographische Signale und EKG-Signale kombiniert erfasst und ausgewertet werden. Die Auswertungseinheit der Messvorrichtung kann dann mit Vorteil zur Auswertung des zeitlichen Verlaufs der Volumenpulssignale und der EKG-Signale eingerichtet sein. Mittels einer geeigneten Programmsteuerung ist die Auswertungseinheit der erfindungsgemäßen Messvorrichtung dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund ihrer Programmsteuerung dazu in der Lage, die Maxima in dem Volumenpulssignal zu erkennen. Anhand der Maxima in dem Volumenpulssignal ist der Zeitpunkt des Eintreffens einer bei einem Herzschlag ausgelösten Pulswelle an dem von der Messvorrichtung erfassten peripheren Messort feststellbar. Somit kann schließlich der zeitliche Abstand zwischen einer R-Zacke in dem EKG-Signal und dem darauf folgenden Maximum in dem Volumenpulssignal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Eine exakte Bestimmung des Blutdrucks aus der Pulswellengeschwindigkeit ist allerdings nicht möglich, es können nur Tendenzen angegeben werden. Weiterhin ist die Pulswellengeschwindigkeit von der Dichte des Blutes und insbesondere von der Elastizität der Blutgefäßwandungen (beispielsweise der Aorta) abhängig. Aus der Elastizität der Blutgefäße kann wiederum auf eine ggf. vorliegende Arteriosklerose geschlossen werden. Es können in diese Auswertung auch die Absolutwerte der Herzfrequenz, die Herzfrequenzvariabilität und entsprechende Arrhythmien des Herzens einbezogen werden. So können automatisch Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpulssignals feststellbar.

Durch die Kombination der Auswertung des EKG-Signals und des Volumenpulssignals bei der automatischen Auswertung ist die erfindungsgemäße Messvorrichtung zur funktionalen Bewertung des Gefäßsystems des Patienten selbsttätig in der Lage. Auf der Grundlage der automatisch ausgewerteten Signale kann die erfindungsgemäße Vorrichtung den (globalen) kardiovaskulären Zustand oder allgemein die Fitness des Benutzers grob einschätzen und bei Anzeichen einer Arteriosklerose oder sonstiger Herz-Kreislauf-Probleme ein entsprechendes Warnsignal oder einen leicht interpretierbaren Fitness- oder Risikoindikator für den Benutzer der Vorrichtung erzeugen. Somit kann die erfindungsgemäße Messvorrichtung vorteilhaft zur Selbstdiagnose von Herz-Kreislauf-Erkrankungen verwendet werden.

Besonders vorteilhaft ist die erfindungsgemäße Kombination der vorgenannten Messverfahren, nämlich der Oximetrie, der bioelektrischen Impedanzmessung und der Wärmemessung. Mittels der Auswertungseinheit der Vorrichtung können sämtliche Messsignale ausgewertet werden, um daraus die arterielle, die kapillare und die venöse Sauerstoffsättigung und daraus wiederum die lokale Stoffwechselaktivität zu bestimmen. Dadurch wird eine hohe Effektivität und Zuverlässigkeit bei der Erkennung und Lokalisierung von pathologischen Veränderungen erreicht. Diese wird durch zusätzliche Berücksichtigung des lokalen Hautkolorits noch verbessert. Die zusätzliche EKG-Messung erlaubt, wie oben ausgeführt, Aussagen bezüglich des Status des Herz-Kreislauf-Systems des Benutzers. Sämtliche Parameter können mit Vorteil zu einem globalen Index zusammengefasst werden, der für den Benutzer leicht interpretierbar ist und ihm einen direkten und fundierten Hinweis auf seinen allgemeinen Gesundheitszustand gibt.

Die Kombination der verschiedenen Messverfahren, die in der erfindungsgemäßen Messvorrichtung, wie oben beschrieben, zusammengefasst sind, ist weiterhin vorteilhaft, weil dadurch eine nicht-invasive Messung der Glukosekonzentration möglich ist, wie im Folgenden erläutert wird:

Die erfindungsgemäße Messvorrichtung dient zur Messung und zur Auswertung von Daten, die durch den Stoffwechsel beeinflusst werden. Es leuchtet unmittelbar ein, dass dabei der Energiehaushalt und die Zusammensetzung der von einem Benutzer der Messvorrichtung aufgenommenen Nahrung eine große Rolle spielen. Die Nährstoffe, die am Stoffwechsel beteiligt sind, sind bekanntlich im Wesentlichen Kohlenhydrate, Fette und Eiweiße. Kohlenhydrate werden zur weiteren Verarbeitung in Glukose, Eiweiße in Aminosäuren, und Fette in Fettsäuren umgewandelt. Die Energieträger werden dann wiederum in den Zellen des Körpergewebes zusammen mit Sauerstoff unter Abgabe von Energie zu ATP (Adenosintriphosphorsäure) umgewandelt. ATP ist der eigentliche körpereigene Energieträger.

Die Verwendung von Glukose zur Erzeugung von ATP ist bevorzugt. Wenn die Erzeugung von ATP aus Glukose jedoch (z. B. wegen eines Mangels an Insulin) gehemmt ist, findet stattdessen eine verstärkte Fettsäure-Oxidation statt. Der Sauerstoffverbrauch ist bei diesem Prozess allerdings ein anderer.

Die Reaktion des Metabolismus des menschlichen Körpers auf eine Nahrungsaufnahme hängt, wie zuvor erwähnt, von der Zusammensetzung der Nahrung charakteristisch ab. So reagiert beispielsweise das vaskuläre System des Körpers in Abhängigkeit davon, wie viel Energie der Körper zur Verdauung der aufgenommenen Speisen benötigt. Anhand der mittels der erfindungsgemäßen Messvorrichtung bestimmbaren Pulswellengeschwindigkeit sowie auch anhand der Blutdruckamplitude und des Pulses lässt sich die Reaktion des Körpers auf die Nahrungsaufnahme bestimmen. Hierzu ist zweckmäßigerweise die Auswertungseinheit der erfindungsgemäßen Messvorrichtung zur Auswertung des zeitlichen Verlaufs der Pulswellengeschwindigkeit und zur Ermittlung der Zusammensetzung von einem Benutzer der Messvorrichtung aufgenommener Nahrung anhand des zeitlichen Verlaufs der Pulswellengeschwindigkeit ab dem Zeitpunkt der Nahrungsaufnahme eingerichtet. Die Pulswellengeschwindigkeit, sowie auch die Blutdruckamplitude und der Puls ändern sich, sobald die Nahrungsaufnahme beginnt. Die Maxima und die jeweiligen Zeitpunkte der Maxima sind dabei beeinflusst durch die Nahrungszusammensetzung. Der Verlauf und die absolute Höhe von Pulswellengeschwindigkeit, Blutdruckamplitude und Puls können herangezogen werden, um mittels der Auswertungseinheit der erfindungsgemäßen Messvorrichtung die Zusammensetzung der aufgenommenen Nahrung zu bestimmen.

Der Metabolismus des menschlichen Körpers ist im Normalzustand, d. h. in Ruhe und in der so genannten thermoneutralen Zone, im Wesentlichen durch den Glukosehaushalt bestimmt. Daher kann die Glukosekonzentration in den Zellen des Körpergewebes in diesen Normalzustand als reine Funktion der Wärmeproduktion und des Sauerstoffverbrauchs beschrieben werden. Es gilt:

$$[Glu] = f_1(\Delta T, VO_2) \, ,$$

wobei [Glu] für die Glukosekonzentration steht. Die Wärmeproduktion $\Delta T$ kann mittels des Wärmesensors der erfindungsgemäßen Messvorrichtung z.B. aus der Differenz zwischen der arteriellen Temperatur und der Temperatur, welche die Hautoberfläche bei perfekter thermischer Isolierung erreichen würde, bestimmt werden ($\Delta T = T_\infty - T_{Arterie}$). $f_1 (\Delta T, VO_2)$ gibt die funktionale Abhängigkeit der Glukosekonzentration von der Wärmeproduktion und vom Sauerstoffverbrauch an. Der Sauerstoffverbrauch ergibt sich, wie oben beschrieben, aus dem Unterschied zwischen venöser und arterieller Sauerstoffsättigung und der Durchblutung. Zur Bestimmung der Glukosekonzentration während bzw. direkt nach der Nahrungsaufnahme muss jedoch ein Korrekturtherm berücksichtigt werden, der den Anteil des Fettstoffwechsels am Energiehaushalt wiedergibt. Es gilt dann:

$$[Glu] = f_1(\Delta T, VO_2) + X * f_2(\Delta T, VO_2)$$

X ist ein Faktor, der nach der Nahrungsaufnahme negativ ist. Dabei hängt X von der Zusammensetzung der aufgenommenen Nahrung ab. Insbesondere ist X davon abhängig, in welchem Verhältnis Fett und Kohlenhydrate am Metabolismus beteiligt sind. Der Faktor X lässt sich, wie oben beschrieben, anhand des zeitlichen Verlaufs der Pulswellengeschwindigkeit bestimmen. X ist 0, wenn reine Kohlenhydrate oder direkt Glukose aufgenommen werden. Der Betrag von X steigt an, je größer der Anteil von Fett an der aufgenommenen Nahrung ist. Zur Bestimmung des Korrekturfaktors X aus dem zeitlichen Verlauf der Pulswellengeschwindigkeit, der Blutdruckamplitude und/oder des Pulses wird normalerweise eine Eichung der erfindungsgemäßen Messvorrichtung zur Anpassung an den jeweiligen Benutzer der Vorrichtung erforderlich sein. $f_2 (\Delta T, VO_2)$ gibt für den Fettstoffwechsel die funktionale Abhängigkeit der Glukosekonzentration von der Wärmeproduktion und vom Sauerstoffverbrauch an.

Die Auswertungseinheit der erfindungsgemäßen Messvorrichtung kann somit zur Bestimmung der lokalen Glukosekonzentration z.B. aus dem lokalen Sauerstoffverbrauch und der lokalen Wärmeproduktion eingerichtet sein. Hierzu muss die Messvorrichtung die geeigneten Messmodalitäten aufweisen. Die Ermittlung des Sauerstoffverbrauchs, kann, wie oben erläutert, durch die Kombination der Oximetrie mit der bioelektrischen Impedanzmessung erfolgen. Zur Ermittlung der Wärmeproduktion ist dann noch zusätzlich ein geeigneter Wärmesensor erforderlich. Um schließlich die Glukosekonzentration nach dem oben angegebenen funktionalen Zusammenhang berechnen zu können, muss noch der Korrekturfaktor X, beispielsweise aus dem zeitlichen Verlauf der Pulswellengeschwindigkeit, ermittelt werden. Dies kann, wie ebenfalls oben erläutert, durch kombinierte Messung von EKG-Signalen und plethysmographischen Signalen erfolgen. Zur Bestimmung der Glukosekonzentration sind also zweckmäßigerweise in der erfindungsgemäßen Messvor-

richtung ein Pulsoximeter, eine EKG-Einheit, eine bioelektrische Impedanzmesseinheit sowie ein Wärmesensor kombiniert.

Die zuvor skizzierte Methode erlaubt zunächst nur eine Bestimmung der intrazellulären Glukosekonzentration. Mit der Blutglukosekonzentration besteht vereinfacht der folgende Zusammenhang:

$$[Glu]_{Zelle} = a + b * \ln (c * [Glu]_{Blut})$$

Die Konstanten a, b und c hängen von der individuellen Physiologie des Benutzers der Messvorrichtung ab. Somit kann die Auswertungseinheit der erfindungsgemäßen Messvorrichtung weiterhin eingerichtet sein zur Bestimmung des Blutglukosespiegels aus der lokalen Glukosekonzentration, wobei von der Physiologie des Benutzers der Messvorrichtung abhängige Parameter berücksichtigt werden müssen. Diese Parameter können durch entsprechende Eichung bestimmt werden, beispielsweise durch Vergleich mit in herkömmlicher Weise invasiv bestimmten Blutglukosewerten.

Die erfindungsgemäße Vorrichtung kann weiterhin eine Datenübertragungsschnittstelle umfassen zur Übertragung der mittels der Auswertungseinheit ermittelten Parameter an einen Personalcomputer (des Arztes), beispielsweise über das Internet, oder an ein anderes Gerät. Hierbei kann es sich um eine übliche drahtgebundene oder auch um eine drahtlose Schnittstelle (beispielsweise nach dem DECT-, GSM-, UMTS- oder Bluetooth-Standard) handeln. Eine Datenübertragung über Infrarotdatenkommunikation oder Ultraschall ist ebenfalls denkbar.

Eine besonders sinnvolle Ausgestaltung der erfindungsgemäßen Messvorrichtung ergibt sich, wenn diese eine Speichereinheit zur Speicherung der mittels der Auswertungseinheit ermittelten Parameter aufweist. Mittels der Speichereinheit können einerseits der Verlauf einer Erkrankung und andererseits die Effekte einer entsprechenden Therapie verfolgt und dokumentiert werden. Andererseits können die in der Speichereinheit abgespeicherten Daten vom behandelnden Arzt ausgelesen und ausgewertet werden, um eine detaillierte Zustandsdiagnostik durch den Arzt zu ermöglichen. Sinnvoll ist es weiterhin, wenn die erfindungsgemäße Vorrichtung eine Diagnoseeinheit zur Bewertung der mittels der Auswertungseinheit ermittelten Parameter und zur Registrierung von Veränderungen der Parameter in Abhängigkeit vom Messort und von der Messzeit aufweist. Demgemäß hat die erfindungsgemäße Vorrichtung einen modularen Aufbau. Die Auswertungseinheit ist lediglich dafür zuständig, die erfassten Signale auszuwerten, um daraus die für die Diagnostik erforderlichen Parameter in der oben beschriebenen Art und Weise zu bestimmen. Diese Parameter werden dann von der Diagnoseeinheit weiter verarbeitet, um daraus Rückschlüsse bezüglich etwaiger Erkrankungen zu ziehen. Die Diagnoseeinheit ist auch dafür zuständig, insbesondere bei Verwendung der Messvorrichtung zur Selbstdiagnose durch einen Benutzer, das Vorliegen einer Erkrankung automatisch zu erkennen und gegebenenfalls ein entsprechendes Warnsignal für den Benutzer zu erzeugen.

Sinnvollerweise ist also die Diagnoseeinheit der erfindungsgemäßen Messvorrichtung zur Bestimmung des Status des Herz-Kreislauf-Systems aus den mittels der Auswertungseinheit ermittelten Parametern eingerichtet. Gemäß einer besonders vorteilhaften Ausgestaltung der Erfindung ist die Diagnoseeinheit außerdem zur Berechnung eines globalen Fitnessindex auf der Basis des Status des Herz-Kreislauf-Systems und den (mittels bioelektrischer Impedanzmessung erfassten) globalen Gewebeparametern eingerichtet. Somit können die globalen Gewebeparameter genutzt werden, um den globalen Fitnessindex zu erhalten, der besonders aufschlussreich Auskunft über den momentanen Gesundheitszustand des Benutzers gibt. Zur Bestimmung des globalen Fitnessindex können sämtliche erfassten Messwerte des Benutzers einbezogen werden. Gegebenenfalls wird eine Mittelung über einen vorgebbaren Zeitraum durchgeführt. Neben den kardiovaskulären Messwerten und den globalen Gewebeparametern (globaler Fettgehalt, globaler Wassergehalt) können auch die lokalen Gewebeparameter sowie die lokalen metabolischen Parameter (z. B. lokaler Sauerstoffverbrauch) mit berücksichtigt werden. Das Ergebnis ist dann der globale Fitnessindex als einzelner Wert, der für den Benutzer der Messvorrichtung besonders einfach interpretierbar ist. Gemäß einer besonders vorteilhaften Ausgestaltung sind sämtliche Komponenten der erfindungsgemäßen Messvorrichtung in einem gemeinsamen Gehäuse angeordnet. Dabei weist die Vorrichtung an einem Ende des Gehäuses einen Messkopf mit den benötigten Messsensoren auf. Auf diese Weise ist die Messvorrichtung handführbar und kann, entweder vom Benutzer selbst oder vom behandelnden Arzt, genutzt werden, um den gesamten Körper systematisch auf krankhafte Veränderungen hin zu untersuchen. In das Gehäuse sollte eine Anzeigeeinheit integriert sein, mittels welcher die lokale Sauerstoffkonzentration des Blutes und/oder die gemäß der Erfindung ermittelten lokalen metabolischen Parameter für den Arzt oder den Benutzer anzeigbar sind.

Der Messkopf der erfindungsgemäßen Messvorrichtung umfasst sinnvollerweise wenigstens eine optische Strahlungsquelle und wenigstens zwei Sensoren, die in unterschiedlichem Abstand zur Strahlungsquelle an dem Messkopf angeordnet sind. Mittels der Strahlungsquelle wird Licht bei verschiedenen Lichtwellenlängen erzeugt. Mittels der Sensoren wird die vom untersuchten Körpergewebe zurückgestreute Strahlung gemessen, um aus der Strahlungsabsorption auf die Sauerstoffkonzentration zurückzuschließen. Die in unterschiedlichem Abstand zur Strahlungsquelle angeordneten Sensoren erlauben es, - wie oben erläutert - die Strahlungsabsorption in unterschiedlich tiefen Gewebeschichten zu untersuchen. Dies ermöglicht es, die Sauerstoffkonzentration im Gewebe von der arteriellen Sauerstoffkonzentration zu unterscheiden.

Weiterhin kann der Messkopf der erfindungsgemäßen Messvorrichtung Elektroden zur bioelektrischen Impedanzmessung und zur EKG-Messung umfassen. Für eine Zweipunktmessung kann eine weitere EKG-Elektrode in das Gehäuse der Messvorrichtung integriert sein. Diese weitere EKG-Elektrode kann gleichzeitig zur bioelektrischen Impedanzmessung, nämlich zur Messung von globalen Gewebeparametern, wie globaler Fettgehalt und/oder globaler Wassergehalt, genutzt werden. Die Anordnung der Elektroden ist zweckmäßigerweise so gewählt, dass eine bioelektrische Impedanzmessung von einem Arm des Benutzers zum anderen Arm möglich ist. Außerdem kann in den Messkopf wenigstens ein Wärmesensor zur Bestimmung der über die Hautoberfläche abgegebenen Wärme integriert sein. Besonders praktisch ist es, wenn bei der erfindungsgemäßen Messvorrichtung die für die verschiedenen Messverfahren (Oximetrie, bioelektrische Impedanzmessung, Wärmemessung, EKG, Messung des Hautkolorits) benötigten Messsensoren in einem einzigen Messkopf zusammengefasst sind. Durch diese Ausgestaltung des Messkopfes ist sichergestellt, dass sämtliche Messwerte gleichzeitig an dem jeweils interessierenden Messort erfasst werden.

Die erfindungsgemäße Messvorrichtung kann miniaturisiert ausgebildet sein und kann in einen am Körper eines Benutzers getragenen Gegenstand, wie beispielsweise eine Armbanduhr, ein Brillengestell oder auch ein Kleidungsstück, integriert sein. Es ist dann eine kontinuierliche Überwachung des Gesundheitszustands möglich.

Die Erfindung wird im Folgenden anhand der Zeichnungen näher erläutert. Es zeigen:

Figur 1    schematische Ansicht einer Messvor- richtung mit vergrößerter Darstellung des Messkopfes (kein Ausführungsbeispiel der Erfindung);

Figur 2    Darstellung der Vorrichtung anhand eines Blockdiagramms;

Figur 3    Blockdiagramm-Darstellung der Oximetrie- einheit der Messvorrichtung gemäß Figur 1;

Figur 4    Blockdiagramm-Darstellung der Wärme- messeinheit;

Figur 5    Blockdiagramm-Darstellung der Impedanz- messeinheit der Messvorrichtung;

Figur 6    Blockdiagramm-Darstellung der EKG-Ein- heit der Messvorrichtung;

Figur 7    Darstellung der Signalauswertung mittels der erfindungsgemäßen Messvorrichtung;

Figur 8    schematische Darstellung einer beispiel- haften Anordnung von Strahlungsquellen, Strahlungssensoren und Elektroden bei der erfindungsgemäßen Messvorrichtung;

Figur 9    Illustration einer Realisierungsmöglichkeit einer erfindungsgemäßen Messvorrichtung mit zwei Strahlungsquellen.

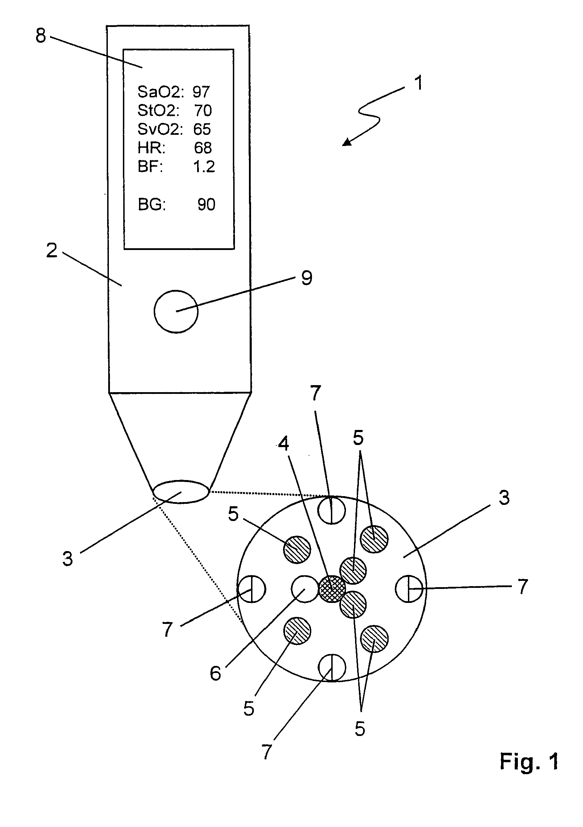

In der Figur 1 ist zu Illustrationszwecken eine nicht erfindungsgemäße Messvorrichtung insgesamt mit der Bezugsziffer 1 bezeichnet. Sämtliche Komponenten der Vorrichtung sind in einem gemeinsamen Gehäuse 2 untergebracht, so dass das Gerät per Hand an beliebigen Messorten am Körper eines Benutzers zum Einsatz gebracht werden kann. Am vorderen Ende des Gehäuses 2 ist ein Messkopf 3 angeordnet, in den die verschiedenen Messsensoren der Vorrichtung 1 integriert sind. Diese werden bei der Verwendung der Messvorrichtung 1 auf die Hautoberfläche des Benutzers am Messort aufgesetzt. Der Messkopf umfasst eine zentral angeordnete Leuchtdiode 4, die dazu in der Lage ist, Licht bei verschiedenen Wellenlängen zu emittieren. Hierzu können beispielsweise verschiedene lichtemittierende Halbleiterelemente in einem gemeinsamen Gehäuse der Leuchtdiode 4 untergebracht sein. Ebenso denkbar ist die Verwendung von Lichtwellenleitern, um das Licht von verschiedenen Lichtquellen an die Unterseite des Messkopfes 3 zu führen. Des Weiteren umfasst der Messkopf 3 insgesamt sechs Photosensoren 5, die in unterschiedlichem Abstand zu der Lichtquelle 4 angeordnet sind. Zwei der Photosensoren 5 sind direkt neben der Lichtquelle 4 angeordnet. Zwei weitere Sensoren 5 befinden sich in einem mittleren Abstand von der Lichtquelle 4, während die zwei verbleibenden Sensoren 5 in maximalem Abstand zur Lichtquelle 4 angeordnet sind. Die unmittelbar neben der Lichtquelle 4 angeordneten Sensoren 5 empfangen hauptsächlich das an den oberen Hautschichten des Benutzers gestreute Licht. Demgegenüber sind die weiter von der Lichtquelle 4 entfernten Sensoren 5 geeignet, die Lichtabsorption in tieferen Gewebeschichten zu messen. Weiterhin ist unmittelbar neben der Lichtquelle 4 ein Wärmesensor 6 vorgesehen. Dadurch ist gewährleistet, dass die Bestimmung der Durchblutung anhand der Wärmemessung am selben Messort erfolgt wie die optische Messung. Außen am Messkopf 3 sind vier Elektroden 7 zur Messung der lokalen bioelektrischen Impedanz vorgesehen. Die Elektroden sind jeweils zweigeteilt und bestehen aus zwei voneinander elektrisch isolierten, separaten Kontaktflächen. Dabei dient jeweils eine der beiden Kontaktflächen zur Aufprägung eines elektrischen Stromes am Messort, während die andere Kontaktfläche zur Spannungsmessung genutzt wird. Auf diese Weise wird sichergestellt, dass die Messergebnisse nicht von den Kontaktwiderständen der Messelektroden beeinflusst sind. Die vier Elektroden 7 können bei der bioelektrischen Impedanzmessung in verschiedenen Kombinationen genutzt werden, um dadurch die Zuverlässigkeit des Messergebnisses zu optimieren. Zumindest eine der Elektroden 7 wird außerdem als EKG-Elektrode einer EKG-Einheit der Messvorrichtung 1 verwendet. In das Gehäuse 2 der Messvorrichtung 1 ist ein LCD-Display 8 als Anzeigeeinheit integriert. Das LCD-Display 8 dient zur Anzeige der lokalen Sauerstoffkonzentration des Blutes. Dabei werden separat die arterielle ($SaO_2$), die kapillare ($StO_2$) und die venöse ($SvO_2$) Sauerstoffsättigung angezeigt. Angezeigt wird weiterhin die ermittelte Herzfrequenz (HR), der lokal bestimmte Fettgehalt des Gewebes (BF). Schließlich wird noch ein Blutglukosewert (BG) angezeigt. Außerdem ist am Gehäuse 2 ein Ein-/Ausschalter 9 angeordnet, der in üblicher Weise zur Aktivierung bzw. zur Deaktivierung des Gerätes dient. Die Betätigungsfläche des Ein-/Ausschalters 9 bildet außerdem die Kontaktfläche einer weiteren EKG-Elektrode, sodass eine einfache Zweipunktableitung des EKG-Signals des Benutzers der Vorrichtung erfolgen kann. Über die Kontaktfläche ist außerdem eine Arm-zu-Arm-Messung von globalen Gewebeparametern, wie globaler Fettgehalt und/oder globaler Wassergehalt, durch bioelektrische Impedanzmessung möglich.

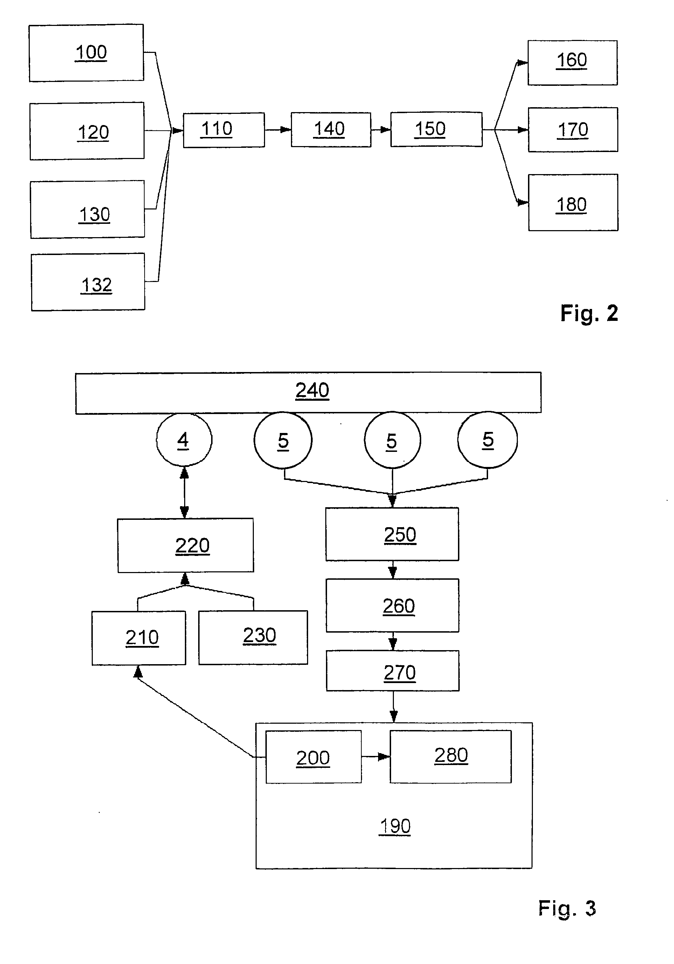

Die Figur 2 zeigt schematisch den Aufbau der Messvorrichtung als Blockdiagramm. Die Vorrichtung 1 umfasst eine optische Messeinheit 100 zur optischen Messung der Sauerstoffkonzentration im Blutgefäßsystem des Körpergewebes am jeweiligen Messort. Die mittels der optischen Messeinheit 100 erfassten oximetrischen und plethysmographische Signale werden einer Analyseeinheit 110 zugeführt. Eine weitere wesentliche Komponente der Vorrichtung 1 ist eine Wärmemesseinheit 120 zur Bestimmung der lokalen Wärmeproduktion. Bei der Wärmemesseinheit 120 handelt es sich um einen speziellen Wärmesensor, welcher die jeweils untersuchte Körperstelle isoliert. Diese Stelle kann somit nur noch Wärme durch den Blutstrom aufnehmen oder abgeben. Daher ist es möglich, durch die zeitaufgelöste Messung der Temperatur die Durchblutung und die Wärmeproduktion zu bestimmen. Bei einer starken Durchblutung erreicht die untersuchte Körperstelle in sehr kurzer Zeit ihre maximale Temperatur. Bei geringer Durchblutung dauert dies länger. Zusätzlich kann über die Extrapolation der gemessenen Temperatur auf die arterielle Temperatur geschlossen werden, da die Temperatur am Ort der Messung nur durch die arterielle Temperatur und durch die lokale Wärmeproduktion bestimmt wird. Auch die mittels der Wärmemesseinheit 120 erfassten Messsignale werden der Analyseeinheit 110 zur Weiterverarbeitung zugeführt. Außerdem umfasst die Messvorrichtung 1 eine Impedanzmesseinheit 130, die zur Erfassung von lokalen Gewebeparametern mittels bioelektrischer Impedanzmessung dient. Die Messsignale der Impedanzmesseinheit 130 werden ebenfalls mittels der Analyseeinheit 110 verarbeitet. Schließlich ist gemäß der Erfindung noch eine EKG-Einheit 132 zur Erfassung eines EKG-Signals vorgesehen. Auch die EKG-Einheit 132 ist zur Verarbeitung der EKG-Signale mit der Analyseeinheit 110 verbunden. Der optischen Messeinheit 100 sind die Lichtquelle 4 sowie die Lichtsensoren 5 des in der Figur 1 dargestellten Messkopfes 3 zugeordnet. Die Wärmemesseinheit 120 ist mit dem Wärmesensor 6 verbunden. Die Impedanzmesseinheit 130 erfasst Messsignale über die Elektroden 7 des Messkopfes 3. Die Analyseeinheit 110 führt eine Vorverarbeitung sämtlicher Messsignale durch. Hierzu durchlaufen die Signale ein Bandpass-Filter, um Störungen im Bereich der Netzfrequenz von 50 bzw. 60 Hz herauszufiltern. Des Weiteren werden die Signale einer Rauschunterdrückung unterzogen. Nach Passieren der Analyseeinheit 110 gelangen die aufbereiteten Signale der optischen Messeinheit 100, der Wärmemesseinheit 120, der Impedanz-Messeinheit 130 und der EKG-Einheit 132 in eine Auswertungseinheit 140. Die Auswertungseinheit 140 ist dafür zuständig, aus den Messsignalen die für die Diagnose wesentlichen Parameter zu berechnen. Aus den zeitabhängig aufgenommenen Messsignalen der Impedanzmesseinheit 130 wird zunächst die Zusammensetzung des untersuchten Körpergewebes (Wassergehalt, Fettgehalt usw.) berechnet. Aus den Signalen der optischen Messeinheit 100 wird die arterielle Sauerstoffsättigung und - unter Zugrundelegung der zuvor auf der Basis der Impedanzmessung ermittelten Gewebeparameter - die kapillare Sauerstoffsättigung berechnet. Weiterhin werden aus den Messsignalen der Wärmemesseinheit 120 und aus den plethysmographischen Daten, die aus der zeitabhängigen Impedanzmessung ableitbar sind, die Durchblutung und die arterielle Temperatur bestimmt. Aus den Signalen der EKG-Einheit 132 und denjenigen der optischen Messeinheit 100 wird die Pulswellengeschwindigkeit bestimmt. Schließlich werden mittels der Auswertungseinheit 140 aus den Ergebnissen sämtlicher zuvor durchgeführter Berechnungen die venöse Sauerstoffsättigung, und daraus weitere metabolische Parameter, insbesondere der lokale Sauerstoffverbrauch und die Glukosekonzentration am Messort berechnet. Die Berechnungsergebnisse werden mittels einer Diagnoseeinheit 150 interpretiert. Die Diagnoseeinheit 150 dient zur Bewertung der mittels der Auswertungseinheit 140 berechneten lokalen metabolischen Parameter. Die Auswertungseinheit 140 und die Diagnoseeinheit 150 sind zur Anzeige der Messresultate mit einer Grafikeinheit 160 verbunden, die ihrerseits die Anzeigeeinheit 8 der Messvorrichtung 1 ansteuert. Die gewonnenen Daten sind in einer Speichereinheit 170 speicherbar, und zwar unter gleichzeitiger Speicherung des Datums und der Uhrzeit der jeweiligen Messung. Außerdem ist eine Schnittstelleneinheit 180 vorgesehen, die zur Verbindung der Messvorrichtung 1 mit einem Computer oder einem anderen Kommunikationsgerät dient. Über die Schnittstelleneinheit 180 können sämtliche Daten und Parameter, insbesondere auch die in der Speichereinheit 170 gespeicherten Daten und Parameter, an einen nicht näher dargestellten PC eines behandelnden Arztes übertragen werden. Dort können die Daten detaillierter analysiert werden. Insbesondere können über einen längeren Zeitraum mit der Vorrichtung 1 aufgenommene Daten und Parameter auf Veränderungen hin untersucht werden, um daraus Schlussfolgerungen hinsichtlich der Entwicklung einer bestehenden Erkrankung ableiten zu können. Außerdem besteht die Möglichkeit, die erfindungsgemäße Messvorrichtung 1 als bloße Messdatenerfassungs- und -sendeeinheit zu benutzen, wobei die aufgenommenen Signale direkt an den PC des Arztes übertragen werden. Mittels des PCs können die entsprechenden Auswertungen und Berechnungen dann gegebenenfalls schneller und komfortabler durchgeführt werden.

Die Figur 3 illustriert den Aufbau der optischen Messeinheit 100 der Vorrichtung 1. Die optische Messeinheit 100 umfasst einen Mikrokontroller 190. Bestandteil des Mikrokontrollers 190 ist ein Timing-Generator 200. Dieser erzeugt Steuerungssignale, die einer Modulationseinheit 210 zugeführt werden. Dadurch wird die zeitliche Modulation der Lichtemission der Leuchtdiode 4 gesteuert. Die Leuchtdiode 4 ist über eine Regelungseinheit 220 mit der Modulationseinheit 210 verbunden. Die Intensität des von der Leuchtdiode 4 emittierten Lichtes ist außerdem über eine Leistungssteuerungseinheit 230 anpassbar. Die Leuchtdiode 4 ist dazu in der Lage, Licht bei zumindest drei verschiedenen Wellenlängen zu emittieren. Hierzu sind verschiedene Licht emittierende Halbleiterbauelemente in einem einzigen Gehäuse der Leuchtdiode 4 vereinigt. Mittels des Timing-Generators 200 wird die zeitliche Abfolge der Lichtemission bei den verschiedenen Lichtwellenlängen gesteuert. Die in den Messkopf 3 der Vorrichtung 1 integrierten Photosensoren 5 sind ebenso wie die Leuchtdiode 4 mit dem in der Figur 3 schematisch angedeuteten Körpergewebe 240 des Benutzers in Kontakt. In dem Körpergewebe 240 wird das Licht der Leuchtdiode 4 gestreut und entsprechend der Sauerstoffkonzentration des Blutes, das das Gewebe 240 durchströmt, absorbiert. Das gestreute Licht wird von den Photosensoren 5 registriert. Der Photostrom jedes Photosensors 5 wird mittels eines Konverters 250 in eine Spannung umgewandelt, mittels eines Verstärkers 260 verstärkt und mittels eines Analog/Digital-Wandlers 270 in digitale Messsignale umgewandelt. Die Digitalsignale werden sodann einem Demodulator 280 zugeführt, der Bestandteil des Mikrokontrollers 190 ist. Der Demodulator 280 separiert die aufgenommenen Messsignale nach den entsprechenden Lichtwellenlängen und nach den unterschiedlichen Entfernungen zwischen den Photosensoren 5 und der Leuchtdiode 4. Schließlich werden die Signale an die Analyseeinheit 110 weitergegeben.

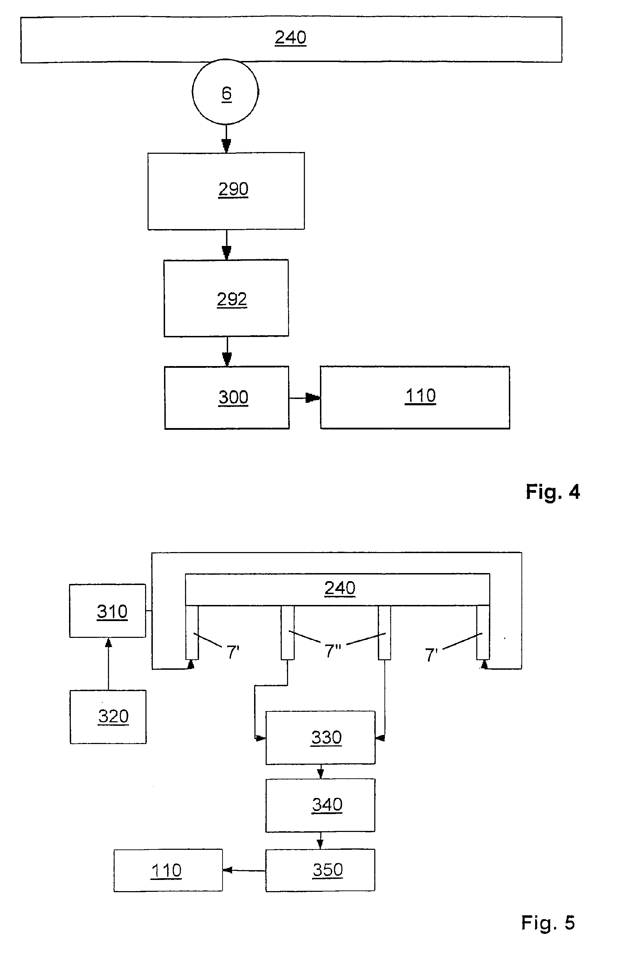

Anhand der Figur 4 wird der Aufbau der Wärmemesseinheit 120 der Messvorrichtung 1 erläutert. Der Wärmesensor 6, der mit dem Körpergewebe 240 in Berührung ist, weist mehrere nicht näher dargestellte Temperaturmesselemente sowie ein wärmeleitendes Element auf. Sobald der Sensor 6 mit dem Gewebe 240 in Kontakt kommt, beginnt ein Wärmeaustausch. Mittels der Temperaturmesselemente wird die Temperatur an verschiedenen Stellen an dem wärmeleitenden Element des Sensors 6 gemessen. Hieraus kann die in dem Gewebe 240 lokal produzierte Wärme (orts-, zeit- und tiefenaufgelöst) bestimmt werden. Die mittels der Temperaturmesselemente erfassten Signale durchlaufen einen Impedanzwandler 290 sowie einen Verstärker 292 und werden mittels eines Analog/Digital-Wandlers 300 digitalisiert. Die digitalen Messsignale werden sodann der Analyseeinheit 110 zur weiteren Verarbeitung zugeführt. Ein geeigneter Wärmesensor 6 ist beispielsweise in der Veröffentlichung von Ok Kyung Cho et al. (Ok Kyung Cho, Yoon Ok Kim, Hiroshi Mitsumaki, Katsuhiko Kuwa, "Noninvasive Measurement of Glucose by Metabolic Heat Conformation Method", Clinical Chemistry 50, 2004, Nr. 10, S. 1894 bis 1898) beschrieben.

In der Figur 5 ist der Aufbau der Impedanzmesseinheit 130 der Messvorrichtung 1 dargestellt. Die Impedanzmesseinheit 130 umfasst mehrere Elektroden 7. Über Kontaktflächen 7' wird dem untersuchten Körpergewebe 240 ein Wechselstrom aufgeprägt, der mittels einer Stromquelle 310 erzeugt wird. Die Stromquelle 310 wird von einem Sinusgenerator 320 angesteuert. Die Frequenz des Wechselstroms variiert zwischen 20 kHz und 100 kHz. Über Kontaktflächen 7" wird eine Spannung als Messsignal am Körpergewebe 240 abgegriffen. Aus dem Verhältnis der gemessenen Spannung zu dem aufgeprägten Strom kann auf die lokale Impedanz des Körpergewebes 240 zurückgeschlossen werden. Hierzu wird die Spannung mittels eines Verstärkers 330 verstärkt und mittels eines Filters 340 gefiltert, um Störsignale zu eliminieren. Wiederum erfolgt eine Digitalisierung mittels eines Analog/Digital-Wandlers 350. Die digitalisierten Messwerte werden wiederum der Analyseeinheit 110 zur weiteren Verarbeitung zugeführt.

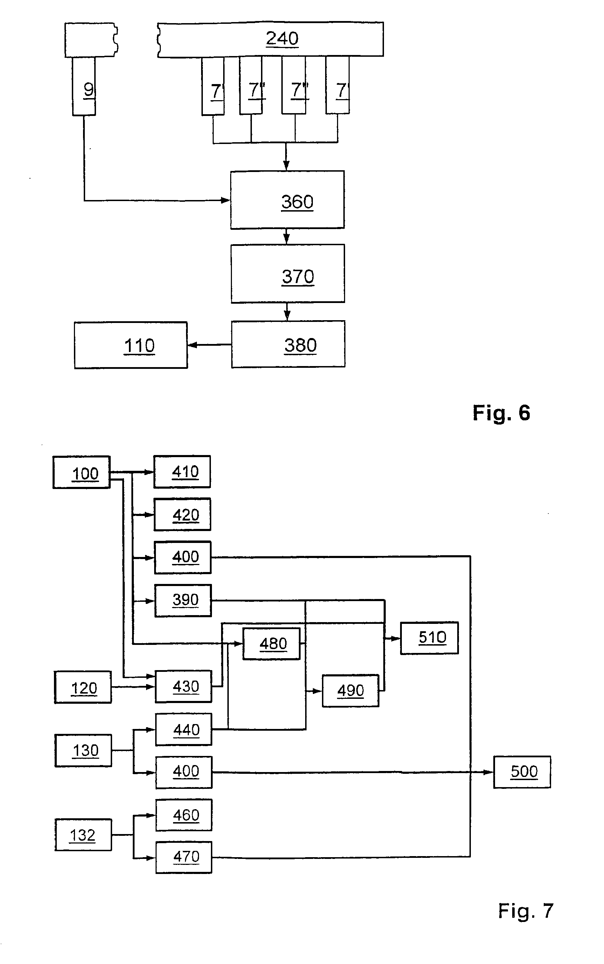

Anhand der Figur 6 wird der Aufbau der EKG-Einheit 132 der Messvorrichtung 1 veranschaulicht. Die EKG-Einheit 132 erfasst ein EKG-Signal über EKG-Elektroden 7' bzw. 7". Es sind dies die Elektroden der Impedanzmesseinheit 130. Die Elektroden 7' und 7" haben also bei dem dargestellten Ausführungsbeispiel eine Doppelfunktion. Für eine brauchbare Zweipunktableitung des EKG-Signals ist eine weitere EKG-Elektrode 9 erforderlich, die in ausreichender räumlicher Entfernung von den Elektroden 7' und 7" mit dem Körper des Benutzers in Kontakt kommt. Die EKG-Elektrode 9 bildet bei dem Ausführungsbeispiel gleichzeitig die Bedienfläche des Ein-/Ausschalters der Messvorrichtung 1. Somit sind sämtliche Elektroden in die Messvorrichtung 1 integriert. Separate, z. B. über Kabel angeschlossene Elektroden sind (für eine einfache Zweipunktableitung des EKG-Signals) nicht zwingend erforderlich. Statt der Bedienfläche des Schalters der Messvorrichtung 1 kann ebenso gut eine zusätzliche Elektrode am Gehäuse 2 der Messvorrichtung 1 angeordnet werden. Das abgeleitete EKG-Signal wird mittels Verstärker 360 und Filter 370 aufbereitet. Nach Passieren eines weiteren Analog/Digital-Wandlers 380 wird das Signal an die Analyseeinheit 110 weitergegeben.

Der Figur 7 ist die Vorgehensweise bei der Ermittlung der physiologischen Parameter gemäß der Erfindung zu entnehmen. Von der optischen Messeinheit 100 werden mittels Pulsoximetrie ein arterieller Sauerstoffsättigungswert 390, mittels Photoplethysmographie ein Volumenpulssignal 400, und mittels Reflexions-/Absorptionsmessung ein Hautkoloritwert 410 geliefert. Außerdem liefert die optische Messeinheit 100 einen Differenzwert 420 zwischen systolischem und diastolischem Volumenpuls. Mittels der Wärmemesseinheit 120 wird die Durchblutung 430 ermittelt. Gleichzeitig kann die Durchblutung 430 auch mittels der optischen Messeinheit 100 gewonnen werden. Die Signale der Impedanzmesseinheit 130 ergeben die lokalen Gewebeparameter 440 und ebenfalls den Volumenpuls 400. Die EKG-Einheit 132 liefert Daten 460 über etwaige Arrhythmien und über die Reizleitung am Herzmuskel. Außerdem ist das eigentliche EKG-Signal 470 mit den Zeitpunkten der Herzaktivitäten verfügbar. Auf der Basis der Gewebeparameter 440 und der arteriellen Sauerstoffsättigung 390 wird die kapillare, d. h. die arteriovenöse Sauerstoffsättigung 480 ermittelt. Daraus wird dann wiederum unter Einbeziehung der Gewebeparameter 440 die venöse Sauerstoffsättigung 490 bestimmt. Aus den Herzschlagzeitpunkten 470 und dem zeitlichen Verlauf der Volumenpulssignale 400 lässt sich die Pulswellengeschwindigkeit 500 ermitteln. Aus diesen Daten kann - wie oben im Einzelnen erläutert - die intrazelluläre Glukosekonzentration des Benutzers bestimmt werden. In die Berechnung der Glukosekonzentration wird der lokale Sauerstoffverbrauch 510 einbezogen, der sich aus der Durchblutung 430, der arteriellen Sauerstoffsättigung 390 und der venösen Sauerstoffsättigung 490 ergibt. Einbezogen wird außerdem die mittels der Wärmemesseinheit 120 ermittelte lokale Wärmeproduktion. Zusätzlich kann ein kardiovaskulärer Index aus den Arrhythmien 460, der Pulswellengeschwindigkeit 500 und der Differenz zwischen systolischem und diastolischem Volumenpuls 420 ermittelt werden. Hierbei können weiterhin der Koloritwert 410 und der Körperfettgehalt 440 einbezogen werden.

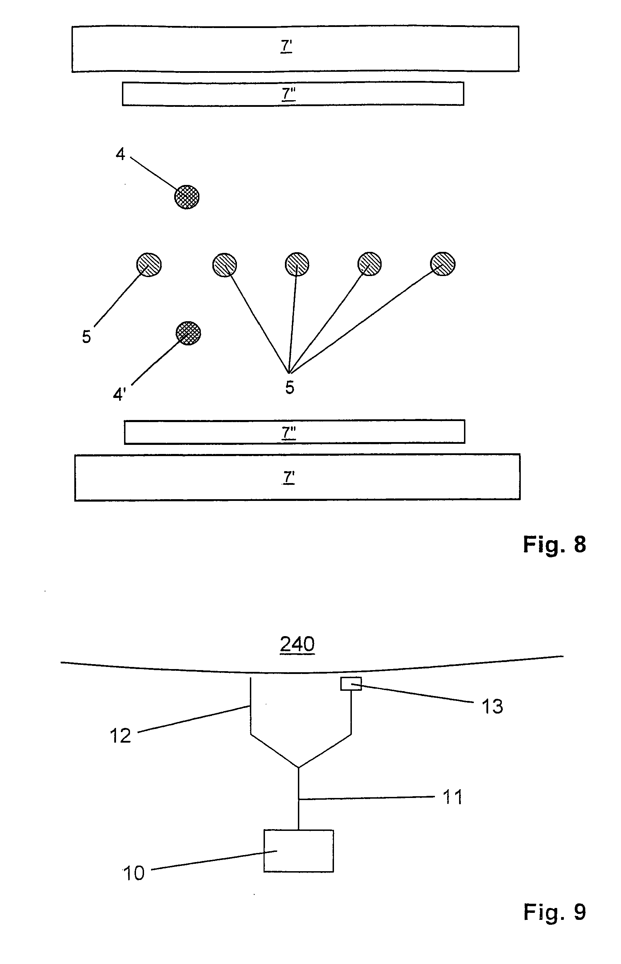

In der Figur 8 ist eine Sensoranordnung gemäß der Erfindung dargestellt. Ähnlich der Darstellung in der Figur 1 zeigt die Figur 8 eine Ansicht der mit der Haut des Benutzers der Vorrichtung in Kontakt zu bringenden Oberfläche des Messkopfes. Die Darstellung in der Figur 8 ist stark vergrößert. Die von den Sensorelementen beanspruchte Fläche kann nur etwa 0,5 bis 2 cm$^2$ betragen.

Bei dem in der Figur 8 dargestellten Ausführungsbeispiel sind zwei Strahlungsquellen 4 und 4' vorgesehen, welche unterschiedliche Volumenbereiche des untersuchten Körpergewebes bestrahlen. Hierzu haben die zwei Strahlungsquellen 4 und 4' unterschiedliche räumliche Abstrahlcharakteristiken, nämlich unterschiedliche Abstrahlwinkel. Bei der Strahlungsquelle 4 handelt es sich um eine Leuchtdiode, während es sich bei der Strahlungsquelle 4' um einen Laser, beispielsweise einen so genannten VCSEL-Laser (engl. "vertical cavity surface emitting laser") handelt. Sowohl die Leuchtdiode 4 als auch der Laser 4' emittieren Licht mit sehr ähnlicher Wellenlänge (z. B. 630 nm und 650 nm), aber mit unterschiedlichen Öffnungswinkeln (z. B. 25° und 55°). Mit der in der Figur 8 dargestellten Anordnung ist - wie oben beschrieben - eine differenzielle Messung von Metabolismus-induzierten Änderungen des Sauerstoffgehalts im Blut möglich. Hierzu muss die Wellenlänge der von den beiden Strahlungsquellen 4 und 4' jeweils emittierten Strahlung in einem Bereich liegen, in welchem das Licht von Oxihämoglobin und Desoxihämoglobin unterschiedlich stark absorbiert wird. Für eine Absolutmessung des Sauerstoffgehalts des Blutes (Sauerstoffsättigung) müssen weitere Strahlungsquellen (in der Figur 8 nicht dargestellt) vorhanden sein, deren Lichtwellenlänge in einem Spektralbereich liegt, in welchem die Lichtabsorption von Oxihämoglobin und Desoxihämoglobin im Wesentlichen gleich ist (so genannter isobektischer Punkt). Das von der Leuchtdiode bzw. von dem Laser emittierte Licht kann mittels entsprechender Lichtleitfasern an die entsprechende Stelle am Messkopf geführt werden. In diesem Fall sind mit den Bezugsziffern 4 und 4' in der Figur 8 die entsprechenden Faserenden dargestellt. Es ist möglich, die Leuchtdiode und den Laser so an die entsprechenden Fasern anzukoppeln, dass sie mit dem gewünschten unterschiedlichen Öffnungswinkel in das zu untersuchende Körpergewebe einstrahlen. Dementsprechend werden mit beiden Strahlungsquellen unterschiedliche Volumina des Körpergewebes untersucht. Aufgrund des größeren Öffnungswinkels ist der Anteil der nicht-durchbluteten Epidermis an dem mittels der Leuchtdiode untersuchten Körpergewebe größer als beim Laser. Das im Körpergewebe gestreute und teilweise absorbierte Licht sowohl der Strahlungsquelle 4 als auch der Strahlungsquelle 4' wird mittels äquidistant zueinander angeordneten Strahlungssensoren 5 detektiert. Hierbei kann es sich um Fotodioden handeln. Vorzugsweise sind die Fotodioden nicht direkt an der Oberfläche des Messkopfes angeordnet. Stattdessen wird das Licht über Lichtleitfasern den Fotodioden zugeführt. Zur Unterscheidung des Lichtes der Strahlungsquelle 4 von dem Licht der Strahlungsquelle 4' können die beiden Lichtquellen 4 und 4' unterschiedlich zeitlich moduliert betrieben werden, wobei die mittels der Sensoren 5 detektierten Signale entsprechend demoduliert werden. Alternativ ist es möglich, die Strahlung der beiden Strahlungsquellen 4 und 4' anhand der unterschiedlichen Wellenlänge zu unterscheiden. Die Strahlungsintensität der von den Strahlungsquellen 4 und 4' emittierten Strahlung wird mit der Weglänge beim Durchgang durch das Körpergewebe geschwächt, wobei der Zusammenhang der Intensitätsschwächung mit der Konzentration der absorbierenden Substanz (oxigeniertes Hämoglobin) durch das bekannte Lambert-Beersche Gesetz gegeben ist. Mittels der in der Figur 8 dargestellten äquidistanten Sensoren 5 können die interessierenden Parameter der Intensitätsschwächung mit großer Genauigkeit bestimmt werden, und zwar getrennt für die von den Strahlungsquellen 4 und 4' jeweils erfassten Volumenbereiche des untersuchten Körpergewebes. Die den verschiedenen Strahlungsquellen 4 und 4' zuzuordnenden Parameter der Intensitätsschwächung können mittels der Auswertungseinheit der erfindungsgemäßen Messvorrichtung zueinander in Beziehung gesetzt werden, um auf diese Weise eine differenzielle Messung durchzuführen. Im einfachsten Fall werden aus den Parametern der Intensitätsschwächung der Strahlung der beiden Strahlungsquellen 4 und 4' jeweils Quotienten berechnet. Aus Änderungen dieser Quotienten kann dann auf Änderungen im Metabolismus zurückgeschlossen werden. Steigt beispielsweise nach der Nahrungsaufnahme der Blutglukosespiegel, gelangt (nach einer gewissen zeitlichen Verzögerung) entsprechend mehr Glukose in die Zellen des Körpergewebes und wird dort umgesetzt. Dabei wird Sauerstoff verbraucht. Diesen Sauerstoff erhalten die Zellen über das Blut. Dabei wird aus dem oxigenierten Hämoglobin durch Abgabe von Sauerstoff desoxigeniertes Hämoglobin. Dementsprechend steigt das Verhältnis von desoxigeniertem Hämoglobin zu oxigeniertem an. Aufgrund der unterschiedlichen Öffnungswinkel der Strahlung der Strahlungsquellen 4 und 4' wirken sich die Änderungen der Hämoglobinkonzentration unterschiedlich auf die jeweilige Intensitätsschwächung aus. Somit können aus den Quotienten der Parameter der Intensitätsschwächung Veränderungen der Hämoglobinkonzentration detektiert werden. Dies ermöglicht es, indirekt auf den Sauerstoffverbrauch zurückzuschließen. Da der Sauerstoffverbrauch seinerseits von dem Blutglukosespiegel abhängt, kann mittels der erläuterten differenziellen Messung der Strahlungsabsorption auch der Blutglukosespiegel ermittelt werden. Als sinnvolle Ergänzung wird parallel zur optischen Messung eine Bioimpedanzanalyse durchgeführt, wozu die in der Figur 8 dargestellten Elektroden 7' und 7" vorgesehen sind. Zweck der Bioimpedanzmessung ist vor allem die Bestimmung der lokalen Durchblutung. Diese kann als weiterer Parameter bei der Bestimmung des Sauerstoffverbrauchs und damit auch des Blutglukosespiegels herangezogen werden. Bei dem in der Figur 8 dargestellten Ausführungsbeispiel sind die Elektroden 7' und 7" auf gegenüberliegenden Seiten der Strahlungsquellen 4 und 4' und der Strahlungssensoren 5 angeordnet, um sicherzustellen, dass von der Bioimpedanzmessung und der optischen Messung derselbe Bereich des untersuchten Körpergewebes erfasst ist.

Die Figur 9 zeigt eine Möglichkeit auf, zwei Strahlungsquellen 4 und 4' mit unterschiedlicher räumlicher Abstrahlcharakteristik einfach und kostengünstig zu realisieren. Hierzu wird ein einzelnes Strahlung emittierendes Element 10, beispielsweise eine Leuchtdiode, benutzt, deren Licht in eine Lichtleitfaser 11 eingekoppelt wird. Die Lichtleitfaser ist an einer geeigneten Stelle in zwei Faserzweige aufgespalten. Über den Faserzweig 12 gelangt das Licht direkt in das untersuchte Körpergewebe 240. In dem anderen Faserzweig ist ein zusätzliches optisches Element 13, beispielsweise eine Linse, vorgesehen, beispielsweise um einen kleineren Abstrahlwinkel zu erzielen. Jeder der in der Figur 9 dargestellten Faserzweige bildet somit eine Strahlungsquelle 4 bzw. 4', wie sie in der Figur 8 dargestellt sind.

Abgesehen von der speziellen Ausgestaltung der Sensorik gemäß den Figuren 8 oder 9 ist das Ausführungsbeispiel der erfindungsgemäßen Messvorrichtung wie oben unter Bezugnahme auf die Figuren 1 bis 7 erläutert ausgebildet.

## Patentansprüche

1.  Messvorrichtung zur nicht-invasiven Bestimmung von physiologischen Parametern, mit wenigstens einer optischen Messeinheit (100) zur Erzeugung von oximetrischen und/oder plethysmographischen Messsignalen, und mit einer Auswertungseinheit (140) zur Verarbeitung der Messsignale, wobei die Auswertungseinheit (140) eingerichtet ist zur Bestimmung wenigstens eines lokalen metabolischen Parameters aus den Signalen der optischen Messeinheit (100),
    **dadurch gekennzeichnet, dass** die optische Messeinheit (100) wenigstens zwei Strahlungsquellen (4, 4') zur Bestrahlung des untersuchten Körpergewebes (240), und wenigstens einen Strahlungssensor (5) zur Detektion der von dem Körpergewebe (240) gestreuten und/oder transmittierten Strahlung aufweist, wobei die Strahlungsquellen (4, 4') unterschiedliche Volumenbereiche des untersuchten Körpergewebes (240) bestrahlen und hierzu unterschiedliche räumliche Abstrahlcharakteristiken haben, wobei die Auswertungseinheit (140) eingerichtet ist zur Bestimmung des lokalen Sauerstoffverbrauchs und/oder des Blutglukosespiegels anhand der Intensitäten der von dem Körpergewebe (240) gestreuten und/oder transmittierten Strahlung der beiden Strahlungsquellen (4, 4').

2.  Messvorrichtung nach Anspruch 1, **gekennzeichnet durch** eine Einheit (120, 130) zur Erfassung von lokalen Gewebeparametern, wie Fettgehalt, Wassergehalt und/oder Durchblutung, wobei die Auswertungseinheit (140) eingerichtet ist zur Bestimmung des wenigstens einen lokalen metabolischen Parameters aus den Signalen der optischen Messeinheit (100) und den Gewebeparametern, wobei die Einheit zur Erfassung von lokalen Gewebeparametern eine bioelektrische Impedanzmesseinheit (130) umfasst.

3.  Messvorrichtung nach einem Anspruch 1 oder 2, **gekennzeichnet durch** eine EKG-Einheit (132) zur Erfassung eines EKG-Signals über zwei oder mehr EKG-Elektroden (7, 9), wobei die Auswertungseinheit (140) zur Auswertung des zeitlichen Verlaufs des EKG-Signals eingerichtet ist.

4.  Messvorrichtung nach einem der Ansprüche 1 bis 3, **gekennzeichnet durch** einen Wärmesensor (6) zur Bestimmung der lokalen Wärmeproduktion, wobei die Auswertungseinheit (140) zur Bestimmung des wenigstens einen lokalen metabolischen Parameters unter Berücksichtigung der Signale des Wärmesensors (6) eingerichtet ist.

5. Messvorrichtung nach Anspruch 4, **dadurch gekennzeichnet, dass** die Auswertungseinheit (140) eingerichtet ist zur Bestimmung der lokalen Glukosekonzentration aus dem lokalen Sauerstoffverbrauch und der lokalen Wärmeproduktion.

6. Messvorrichtung nach einem der Ansprüche 1 bis 5, **dadurch gekennzeichnet, dass** die optische Messeinheit (100) wenigstens zwei Strahlungssensoren (5) zur Detektion der von dem Körpergewebe (240) gestreuten und/oder transmittierten Strahlung aufweist, wobei die Strahlungssensoren (5) in unterschiedlichen Abständen zu den Strahlungsquellen (4, 4') angeordnet sind.

7. Messvorrichtung nach einem der Ansprüche 20 bis 23, **dadurch gekennzeichnet, dass** die Wellenlänge der von den beiden Strahlungsquellen jeweils emittierten Strahlung im Bereich zwischen 600 und 700 nm, vorzugsweise zwischen 630 und 650 nm, liegt.

8. Verfahren zur Erfassung und Auswertung von physiologischen Parametern, insbesondere unter Verwendung einer Messvorrichtung nach einem der Ansprüche 1 bis 7, wobei

- mittels einer optischen Messeinheit (100) oximetrische und/oder plethysmographische Messsignale von Körpergewebe (240) erfasst werden,
- und mittels einer Auswertungseinheit (140) die oximetrischen und/oder plethysmographischen Messsignale verarbeitet werden, und zwar zur Bestimmung des Pulses und/oder der lokalen Sauerstoffkonzentration, wobei mittels der Auswertungseinheit (140) wenigstens ein lokaler metabolischer Parameter aus den oximetrischen Signalen bestimmt wird, **dadurch gekennzeichnet, dass** mittels der optischen Messeinheit (100) unterschiedliche Volumenbereiche des untersuchten Körpergewebes bestrahlt werden, wobei der wenigstens eine lokale metabolische Parameter aus der von dem Körpergewebe (240) in den unterschiedlichen Volumenbereichen gestreuten und/oder transmittierten Strahlung bestimmt wird, wobei die optische Messeinheit (100) wenigstens zwei Strahlungsquellen (4, 4') mit unterschiedlichen räumlichen Abstrahlcharakteristiken umfasst, wobei der lokale Sauerstoffverbrauch und/oder der Blutglukosespiegel anhand der Intensitäten der von dem Körpergewebe (240) gestreuten und/oder transmittierten Strahlung der beiden Strahlungsquellen (4, 4') bestimmt wird.

9. Verfahren nach Anspruch 8, **dadurch gekennzeichnet, dass** lokale Gewebeparameter, wie Fettgehalt, Wassergehalt und/oder Durchblutung, erfasst werden, wobei der wenigstens eine lokale metabolische Parameter aus den oximetrischen Signalen und den lokalen Gewebeparametern bestimmt wird, wobei die lokalen Gewebeparameter mittels bioelektrischer Impedanzmessung erfasst werden.

10. Verfahren nach einem der Ansprüche 8 oder 9, **gekennzeichnet durch** eine zusätzlich zur Erfassung der oximetrischen Messsignale durchgeführte Erfassung eines EKG-Signals.

11. Verfahren nach einem der Ansprüche 8 bis 10, **dadurch gekennzeichnet, dass** lokale Gewebeparameter mittels ortsaufgelöster Wärmemessung erfasst werden.

12. Verfahren nach Anspruch 11, **dadurch gekennzeichnet, dass** die lokale Glukosekonzentration aus dem lokalen Sauerstoffverbrauch und der lokalen Wärmeproduktion bestimmt wird.

13. Verfahren nach Anspruch 12, **dadurch gekennzeichnet, dass** die Bestimmung der lokalen Glukosekonzentration unter Einbeziehung von Daten betreffend die Zusammensetzung von einem Benutzer der Messvorrichtung aufgenommener Nahrung erfolgt.

14. Verfahren nach Anspruch 12 oder 13, **dadurch gekennzeichnet, dass** der Blutglukosespiegel aus der lokalen Glukosekonzentration bestimmt wird, wobei von der Physiologie des Benutzers der Messvorrichtung abhängige Parameter berücksichtigt werden.

SaO2: 97
StO2: 70
SvO2: 65
HR: 68
BF: 1.2

BG: 90

Fig. 1

**Fig. 2**

**Fig. 3**

**Fig. 4**

**Fig. 5**

**Fig. 6**

**Fig. 7**

7'

7"

4

5

4'

5

7"

7'

**Fig. 8**

240

13

12

11

10

**Fig. 9**

EP 2 260 756 A1

Europäisches
Patentamt

European
Patent Office

Office européen
des brevets

# EUROPÄISCHER RECHERCHENBERICHT

**Nummer der Anmeldung**

EP 10 00 9799

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 289 230 B1 (CHAIKEN JOSEPH [US] ET AL) 11. September 2001 (2001-09-11)<br>* Spalte 3, Zeile 6 - Zeile 8 *<br>* Spalte 3, Zeile 32 - Zeile 36 *<br>* Spalte 9, Zeile 9 - Zeile 16 *<br>* Spalte 9, Zeile 44 - Zeile 50 *<br>----- | 1,8 | INV.<br>A61B5/00<br>A61B5/053 |
| A | WO 99/62399 A1 (MASIMO CORP [US])<br>9. Dezember 1999 (1999-12-09)<br>* Seite 6, Zeile 15 - Seite 7, Zeile 5 *<br>* Seite 19, Zeile 33 - Seite 20, Zeile 10 *<br>* Seite 23, Zeile 3 - Zeile 10 *<br>----- | 1-3,5-7,<br>9,10 | |
| A | LEPRETRE PIERRE-MARIE ET AL: "Effect of exercise intensity on relationship between VO2max and cardiac output."<br>MEDICINE AND SCIENCE IN SPORTS AND EXERCISE AUG 2004,<br>Bd. 36, Nr. 8, August 2004 (2004-08),<br>Seiten 1357-1363, XP002428499<br>ISSN: 0195-9131<br>* Seite 1359, Spalte 1, Zeile 4 - Spalte 2, Zeile 2 *<br>----- | 1,2,4,<br>8-10 | |
| A | US 6 790 178 B1 (MAULT JAMES R [US] ET AL)<br>14. September 2004 (2004-09-14)<br>* Spalte 4, Zeile 16 - Zeile 25 *<br>* Spalte 16, Zeile 40 - Zeile 43 *<br>* Spalte 17, Zeile 9 - Zeile 11 *<br>* Spalte 18, Zeile 62 - Zeile 63 *<br>* Spalte 19, Zeile 42 - Zeile 45 *<br>----- | 1-4,8 | RECHERCHIERTE SACHGEBIETE (IPC)<br><br>A61B |
| A | US 2004/034293 A1 (KIMBALL VICTOR E [US])<br>19. Februar 2004 (2004-02-19)<br>* Absätze [0055] - [0060], [0063] *<br>-----<br>-/-- | 1,2,4,7,<br>8 | |

Der vorliegende Recherchenbericht wurde für alle Patentansprüche erstellt

| Recherchenort | Abschlußdatum der Recherche | Prüfer |
|---|---|---|
| Den Haag | 1. November 2010 | Knüpling, Moritz |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

**Nummer der Anmeldung**

EP 10 00 9799

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 5 237 178 A (ROSENTHAL ROBERT D [US] ET AL) 17. August 1993 (1993-08-17) * Spalte 2, Zeile 1 - Zeile 14 * ----- | 1-3,8 | |
| A | EP 1 491 134 A (HITACHI LTD [JP]) 29. Dezember 2004 (2004-12-29) * Absatz [0029] * ----- | 1,2,4,5, 8,9,11, 12,14 | |
| A | US 2004/181132 A1 (ROSENTHAL ROBERT D [US]) 16. September 2004 (2004-09-16) * Absätze [0063] - [0066] * ----- | 1,8,13 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 1. November 2010 | Knüpling, Moritz |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 10 00 9799

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-11-2010

| Im Recherchenbericht angeführtes Patentdokument | | Datum der Veröffentlichung | Mitglied(er) der Patentfamilie | | Datum der Veröffentlichung |
|---|---|---|---|---|---|
| US 6289230 | B1 | 11-09-2001 | KEINE | | |
| WO 9962399 | A1 | 09-12-1999 | AU | 4214199 A | 20-12-1999 |
| | | | CA | 2333062 A1 | 09-12-1999 |
| | | | EP | 1082050 A1 | 14-03-2001 |
| | | | JP | 2002516689 T | 11-06-2002 |
| US 6790178 | B1 | 14-09-2004 | EP | 1217942 A1 | 03-07-2002 |
| | | | WO | 0128416 A1 | 26-04-2001 |
| US 2004034293 | A1 | 19-02-2004 | AU | 2003262620 A1 | 03-03-2004 |
| | | | EP | 1545308 A1 | 29-06-2005 |
| | | | JP | 2005535408 T | 24-11-2005 |
| | | | WO | 2004016170 A1 | 26-02-2004 |
| US 5237178 | A | 17-08-1993 | US | 5362966 A | 08-11-1994 |
| | | | US | 5436455 A | 25-07-1995 |
| EP 1491134 | A | 29-12-2004 | CN | 1573332 A | 02-02-2005 |
| | | | JP | 3566277 B1 | 15-09-2004 |
| | | | JP | 2005013273 A | 20-01-2005 |
| | | | US | 2004260165 A1 | 23-12-2004 |
| US 2004181132 | A1 | 16-09-2004 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0069328 A1
- US 6331162 B
- US 4960126 A
- US 6714814 B
- US 4928014 A

**In der Beschreibung aufgeführte Nicht-Patentliteratur**

- **Meir Nitzan ; Boris Khanokh.** Infrared Radiometry of Thermally Insulated Skin for the Assessment of Skin Blood Flow. *Optical Engineering,* 1994, vol. 33 (9), 2953-2956
- **Ok Kyung Cho ; Yoon Ok Kim ; Hiroshi Mitsumaki ; Katsuhiko Kuwa.** Noninvasive Measurement of Glucose by Metabolic Heat Conformation Method. *Clinical Chemistry,* 2004, vol. 50 (10), 1894-1898